(19) Europäisches Patentamt
European Patent Office
Office européen des brevets

(11) Publication number: **0 027 155**
**B1**

(12) **EUROPEAN PATENT SPECIFICATION**

(45) Date of publication of patent specification: **31.08.83**

(51) Int. Cl.³: **C 07 C 17/34,** C 07 C 21/24, C 07 C 25/28

(21) Application number: **79302176.7**

(22) Date of filing: **10.10.79**

(54) **Process for dehydrohalogenating (polyhaloalkyl)benzenes.**

(43) Date of publication of application:
**22.04.81 Bulletin 81/16**

(45) Publication of the grant of the patent:
**31.08.83 Bulletin 83/35**

(84) Designated Contracting States:
**BE CH DE FR IT NL SE**

(56) References cited:
**GB - A - 1 231 179**
**US - A - 2 874 197**
**US - A - 3 391 203**
**US - A - 3 424 803**
**US - A - 3 454 657**
**US - A - 3 634 485**

(73) Proprietor: **THE DOW CHEMICAL COMPANY**
**Dow Center 2030 Abbott Road Post Office Box 1967**
**Midland Michigan 48640 (US)**

(72) Inventor: **Markley, Lowell Dean**
**1307 Wildwood, Midland**
**Midland, Michigan (US)**
Inventor: **Wang, Chun-Shan**
**1906 Burlington, Midland**
**Midland, Michigan (US)**
Inventor: **McGregor, Stanley Dane**
**1424 Jay Street, Midland**
**Midland, Michigan (US)**
Inventor: **Nestrick, Terry John**
**508, George Street, Midland**
**Midland, Michigan (US)**

(74) Representative: **Allard, Susan Joyce et al,**
**BOULT, WADE & TENNANT 27 Furnival street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 027 155 B1**

Process for dehydrohalogenating (polyhaloalkyl) benzenes

This invention relates to processes for preparing (haloalkenyl) benzenes including (polyhaloalkenyl)benzenes.

α-(1-Haloalkyl)styrenes are useful as parasiticides and insecticides. They are also useful intermediates in the manufacture of other biologically active compounds. Such compounds may be prepared by the process disclosed in U.S. Patent No. 3454657 in which a halogenated organic compound is reacted with an α-methylstyrene in the presence of a catalyst which comprises an organic amine and a copper-containing material.

U.S. Patent No. 3424803 discloses a process for the production of 1-nitro-3-(3,3,3-trichloro-1-methylenepropyl)benzene in which 1,1-dichloroethyl-3-nitrobenzene is reacted with vinylidene chloride and the resulting product subjected to dehydrohalogenation. The dehydrohalogenation is carried out using an acid catalyst such as Lewis acid.

We have now found that (haloalkyl)benzenes and other (haloalkenyl)arenes can be produced in high yield in a dehydrohalogenation process in which certain selected Lewis acid catalysts are used.

Accordingly, the present invention provides a process for the dehydrohalogenation of a (polyhaloalkyl)arene wherein in the polyhaloalkyl one halogen is a benzylic halogen and at least one halogen is a non-benzylic halogen which process comprises contacting the (polyhaloalkyl)arene with a catalytic amount of a suitably active Lewis acid which is either (i) unsupported and is a halide of titanium which has been treated with water or a halide of antimony, or (ii) deposited upon an inert solid particulate support, the reaction being carried out at a temperature of below 100°C, to form a (haloalkenyl)arene.

Surprisingly, the benzylic halogen of the polyhaloalkyl group is selectively eliminated to form the desired (haloalkenyl)arene while the non-benzylic aliphatic halogen(s) are left undisturbed. The (haloalkenyl)benzenes produced in the practice of this invention are useful as biologically active compounds as described hereinbefore and as intermediates in the preparation of other biologically active compounds.

By a (polyhaloalkyl)arene is meant an aromatic compound in which an aromatic ring bears at least one polyhaloalkyl substituent. In the polyhaloalkyl substituent, one halogen is bonded to the alkyl carbon bonded to the aromatic ring (hereinafter called a benzylic halogen) and at least one halogen is bonded to one other alkyl carbon (hereinafter called a non-benzylic halogen). By "arene" is meant an aromatic compound having one or more aromatic rings such as benzene, naphthalene, anthracene as well as substituted arenes. The substituents include halo, nitro, alkyl, alkoxy, alkylthio, aryl, aryloxy, sulfo, carboxy, carboxylate ester, haloalkyl including polyhaloalkyl, haloaryl and other substituent groups that do not interfere with dehydrohalogenation reactions that are catalyzed by Lewis acids. Such substituents are inert in the dehydrohalogenation reactions.

Preferred (polyhaloalkyl)arenes are represented by Formula I:

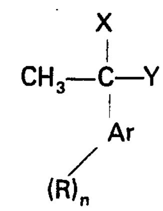 I

wherein Ar is arene, preferably benzene, each R is individually halo, alkyl, haloalkyl including polyhaloalkyl such as $—CX_3$ (e.g., $—CF_3$) and

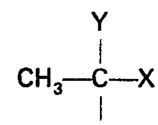

wherein X and Y are as defined herein, aryl, haloaryl, nitro, alkoxy, and other inert monovalent organic radicals, X is halo, Y is haloalkyl or substituted haloalkyl wherein alkyl has from 2 to 3 carbons and the non-halogen substituent or substituents may be, for example, nitro or alkoxy, and n is 0 to the maximum number of remaining available ring positions on Ar. Preferably n is from 0 to 2 when Ar is benzene. More preferably, each R is individually halo such as Cl, Br, or F; alkyl having 1 to 4 carbons such as $—CH_3$; alkoxy such as $—OCH_3$ and others having 1 to 4 carbons; and $—NO_2$. Most preferably each R is individually Cl, Br or $—NO_2$. X is more preferably Cl or Br, most preferably Cl. Y is more preferably haloalkyl represented by the formula:

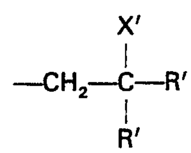

wherein X′ is Cl or Br, each R′ is individually H, halo such as Cl, Br or F, lower alkyl or $—NO_2$. Most preferably Y is $—CH_2CCl_2R'$ wherein R′ is H, Cl, Br, $—CH_3$ or $—C_2H_5$. For example, Y is most preferably $—CH_2CCl_3$, $—CH_2CCl_2Br$, $—CH_2CHCl_2$ and $—CH_2CH_2Cl$.

Examples of especially preferred poly(haloalkyl)arenes include 1,3-dichloro-5-(1,3,3,3-tetrachloro-1-methylpropyl)benzene, 1,3-dichloro - 5 - (1,3,3, - trichloro - 1 - methylpropyl) -

benzene and similar 1,3-dihalo-5-(polyhalobutyl)benzenes. Other preferred (polyhaloalkyl)arenes include 3-chloro-1-(1,3,3,3-tetrachloro-1-methylpropyl)benzene and similar 3-halo-1-(polyhalobutyl)benzenes.

(Polyhaloalkyl)arenes may be prepared by known methods. For example, $\alpha$-methylstyrene or ar-substituted methylstyrene is reacted with a polyhaloalkane such as carbon tetrachloride, bromotrichloromethane, methylene chloride or dichloronitromethane in the presence of an amine and cuprous chloride to produce a desired (polyhaloalkyl)benzene.

Lewis acids which are suitably employed in the practice of this invention are the Lewis acids which either in neat form or when deposited on a support catalyze the elimination of the benzylic halogen from the (polyhaloalkyl)arene via a dehydrohalogenation reaction while essentially all of the non-benzylic halogen substituent(s) of the (polyhaloalkyl)arene remain bonded to the (polyhaloalkyl)arene. Such Lewis acids are stated herein to be suitably active if, during the preferential dehydrohalogenation of essentially all (>95 mole percent) of benzylic halogen of the (polyhaloalkyl)arene, less than 10, preferably less than 5, mole percent of non-benzylic halogen is eliminated.

Lewis acids which, in neat (undiluted) form, are suitably active are (1) the halides of titanium, preferably $TiCl_4$, which have been treated with water and (2) the halides of antimony, preferably antimony pentachloride. Of the neat forms, the titanium tetrahalides which have been treated with from 0.1 to 2 moles of water per mole of the titanium tetrahalide are more preferred, with $TiCl_4$ being treated from 0.25 to 1.75 moles of water per mole of $TiCl_4$ being most preferred.

While most other common Lewis acids such as $AlCl_3$, $FeCl_3$, $SnCl_4$, and the like are not suitably active in neat form, they, as well as most other Lewis acids, are suitably active when deposited on a solid catalyst carrier or support. Examples of such Lewis acids which in supported form are suitably active include the halides, particularly the chlorides and bromides, of such metals as aluminum, iron, zinc, copper, antimony, titanium, bismuth, arsenic, tantalum, vanadium, magnesium, boron and tin; as well as the oxyhalides, oxides, sulfates, phosphates, nitrates and oxylates of such metals. Of the foregoing Lewis acids, the halides, especially the chlorides, of aluminum, antimony, iron and titanium are most preferably employed in combination with a solid particulate catalyst support.

Exemplary solid catalyst supports include silica, silica gel, titania, alumina, silica/alumina, magnesia, asbestos, charcoal, Fuller's earth, diatomaceous earths, vanadia magnesium silicate, bauxite, dausonite, gibbsite, Florida earth, bentonite, kaolin, pipe clay, montmorillonite, and kieselguhr. Advantageously, the catalyst support is in the form of a particulate solid, preferably one having an average particle diameter in the range from 100 to 400 micrometers and a surface area in the range from 80 to 200 square meters per gram. When using the catalyst support, the Lewis acid catalyst component preferably constitutes from 5 to 15 weight percent of the total support catalyst with the remainder being the solid support. The supported Lewis acid is advantageously prepared by slurrying the support in a solution of the desired Lewis acid in an inert organic solvent such as hydrocarbon or halogenated hydrocarbon solvents, e.g., carbon tetrachloride ($CCl_4$), dichloromethane, chloroform, methylchloroform and tetrachloroethylene. Alternatively, both Lewis acid and support can be slurried in an organic diluent which does not dissolve the Lewis acid or the support. In either case, from 0.1 to 3 weight parts of Lewis acid and from 1 to 30 weight parts of solid support are added to 100 weight parts of organic liquid. Advantageously, the resulting slurry is heated at about the reflux temperature, e.g., from 50 to 80°C, of the organic liquid for a time sufficient to cause deposition of the Lewis acid on the support, usually from 10 to 60 minutes. While the concentration of Lewis acid in the total supported catalyst (i.e., the supported Lewis acid) is not particularly critical, the Lewis acid preferably constitutes from 5 to 15 weight percent of the total supported catalyst with the remainder being the solid support. The supported Lewis acid catalysts are preferred over the heat catalysts.

In the practice of this invention, the (polyhaloalkyl)arene is contacted with a catalytic amount of a suitably active Lewis acid under dehydrohalogenation conditions. A catalytic amount is any amount of suitably active Lewis acid which catalyzes the selective dehydrohalogenation of the (polyhaloalkyl)arene such that substantially all of the benzylic halogen thereof is eliminated. Advantageously, such catalytic amounts are within the range from 0.1 to 20 weight percent of suitably active Lewis acid based on the weight of (polyhaloalkyl)arene, preferably from 0.1 to 10 weight percent of the Lewis acid, most preferably from 0.2 to 3 weight percent of the Lewis acid. The foregoing concentrations of catalyst include only the concentration of Lewis acid and not support which is optionally employed. When a supported Lewis acid is employed, the concentration of the support is advantageously in the range from 1 to 100, preferably from 1 to 30, weight percent based on the weight of (polyhaloalkyl)arene.

In addition to the aforementioned starting ingredients, a solvent such as carbon tetrachloride, ethylene dichloride or similar halohydrocarbons is optionally employed. When used, the solvent is present in an amount between 0.5 and 3 liters of solvent per mole of the (polyhaloalkyl)arene.

The reaction is conducted in the liquid phase at a temperature below 100°C, preferably between 25°C and 80°C, and most preferably between 55°C and 80°C. Preferably, the suitably active Lewis acid catalyst is added to a stirred mixture of the (polyhaloalkyl)arene and the optional solvent. It is sometimes desirable, particularly when a neat Lewis acid catalyst is employed, to add the (polyhaloalkyl)arene diluted with solvent to a stirred solution of the catalyst in solvent. Thus, the rate of hydrogen halide evolution is controlled by the slow addition of reactant. When a supported Lewis acid is employed, diluted (polyhaloalkyl)arene is preferably added to a stirred slurry of the supported Lewis acid in organic solvent.

After the (polyhaloalkyl)arene is contacted with catalyst, the reaction begins immediately, as evidenced by evolution of hydrogen halide gas. The reaction is allowed to proceed to completion while agitating the reaction mixture sufficiently to keep the catalyst in suspension. The reaction pressure is not critical and is conveniently atmospheric.

The product of the dehydrohalogenation reaction is primarily a (polyhaloalkenyl)arene wherein the benzylic halogen and hydrogen on an adjacent carbon are eliminated. In embodiments of particular interest, the (polyhaloalkenyl)arene is represented by formula II:

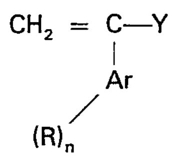

II

wherein R, Y and n are as defined hereinbefore. In preferred embodiments of this invention, the dehydrohalogenation is sufficiently selective such that more than 95 mole percent of benzylic halogen is eliminated, most preferably more than 99 mole percent, and less than 5 mole percent of non-benzylic halogen is eliminated most preferably less than 2 mole percent.

The following examples are given to further illustrate the invention. All percentages in the examples are by weight unless otherwise indicated.

### Example 1

Preparation of 3,5-dichloro-α-methylstyrene according to U.S. Patent 2,816,934

Chlorine gas is bubbled through 129 g of 3,5-dichlorotoluene in the presence of light until no further adsorption occurs. An increase in weight of 83 g results. To the product, weighing 212 g, is added dropwise 400 g of 8 percent fuming sulfuric acid. After being stirred for 30 hours the mixture is poured over cracked ice. The 3,5-dichlorobenzoic acid which precipitated is washed well with water and dried. It weighs 145 g, or 95 percent yield based on the dichlorotoluene. The acid is converted to 3,5-dichlorobenzoyl chloride in 95 percent yield by treating with 125 g thionyl chloride. The chloride, weighing 151 g, is then allowed to react with 150 ml of methyl alcohol and the resulting methyl 3,5-dichlorobenzoate, which when distilled at 120°C—125°C at 9.3 mbar (7 mm) weighs 133 g, or 90 percent of theory. The ester is treated with 2 equivalents of methyl magnesium chloride (125 g), the Grignard complex hydrolyzed, and the product then dehydrated by refluxing with $NaHSO_4$. The 3,5-dichloro-α-methylstyrene obtained weighs 88 g, or 72 percent of theory based on the ester used, and boiled at 109°C—111°C at 16 mbar (12 mm). Its specific gravity is 1.196 and its refractive index is 1.5660, both measured at 25°C.

Addition to $CCl_4$ according to U.S. Patent 3,454,657

The 3,5-dichloro-α-methylstyrene is placed in a 250 ml vessel equipped with stirring means and a heating means. A mixture including 18.7 g (0.1 mole) of 3,5-dichloro-α-methylstyrene, as well as 46.2 g (0.3 mole) of $CCl_4$ and 0.4 g of cuprous chloride is formed with stirring. To the mixture is added 1.6 g (0.016 mole) of cyclohexylamine. The mixture is heated to the reflux temperature of $CCl_4$ and maintained at reflux temperature until completion of the reaction in 30 minutes. The reaction mixture is cooled and filtered, and the solvent is removed under vacuum leaving 31.2 g (91.5 percent yield) of residual product. This residue is recrystallized from hexane to yield essentially pure 1,3-dichloro-5-(1,3,3,3-tetrachloro-1-methylpropyl)benzene exhibiting a melting point of 44.5°—46.5°C.

Dehydrohalogenation with $SbCl_5$

A mixture composed of a 137 g (0.40 mole) portion of the 1,3-dichloro-5-(1,3,3,3-tetrachloro-1-methylpropyl)benzene (DCTCB) obtained above and 250 ml of $CCl_4$ is formed in a 500 ml flask equipped with a stirring and a heating means. With stirring, a 7 g portion (0.023 mole) of $SbCl_5$ is added to the flask and dehydrochlorination (as evidenced by HCl gas) to form a crude product mixture occurs at room temperature. The crude product mixture is heated to reflux (82°C), is held at this temperature for 30 minutes, and is then allowed to cool to a temperature near ambient. To the crude product mixture a 150-ml portion of $CCl_4$ is added and then a 200-ml portion of 3N HCl is added with stirring. An aqueous and an organic layer are formed. The organic and aqueous layers are separated and 200 ml of water is added to the organic layer with stirring. The organic layer is stirred over $Na_2SO_4$ to remove any water remaining. The carbon tetrachloride present in the organic layer is removed under a vacuum leaving 115.8 g of a crude product. Distillation of the crude product yields 100.2 g

(0.33 mole) of product containing at least 95 percent of 3,5-dichloro-$\alpha$-(2,2,2-trichloroethyl)styrene (DCTCS) represented by the structure:

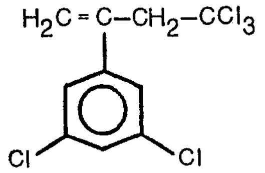

and less than 5 percent of diene represented by the structure:

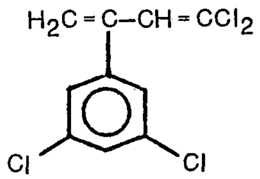

Example 2

Dehydrohalogenation with $H_2O$ Treated $TiCl_4$

A mixture consisting of 5 g (0.0147 mole) of the DCTCB produced according to Example 1, 50 ml of $CCl_4$ and 0.020 ml of water is placed in a 100 ml flask. The mixture is heated to reflux (about 90°C) and 0.173 g (0.000911 mole) of $TiCl_4$ is added to the mixture. Evolution of HCl is noted as the reflux continues for 2 hours. The mixture is cooled to 45°C and 25 ml of concentrated hydrochloric acid is added. An organic layer and an aqueous layer are formed and separated. The organic layer is washed with water. The solvent is then removed from the organic layer in vacuum. The remaining residue which weighs 4.05 g (0.0133 mole) for a 91 percent yield is identified as DCTCS as prepared in Example 1. Analysis by gas-liquid chromatography (GLC) shows the residue to contain 97.5 percent of the aforementioned styrene (DCTCS) and less than 2.5 percent of the diene.

Example 3

Dehydrohalogenation with $AlCl_3$/Silica Gel

A commercially available supported catalyst comprising ~10 percent of $AlCl_3$ and ~90 percent silica gel (~300 micrometers) is added in the amount of 2.5 g to a mixture consisting of 10.6 g (0.031 mole) of DCTCB and 30 ml of $CCl_4$. The resulting mixture is stirred at room temperature and then heated to 70°C for a period of 5 hours. Throughout the reaction period, the mixture is stirred at a rate sufficient to maintain the supported catalyst uniformly dispersed throughout the mixture. The reaction mixture is then filtered to remove solid catalyst. The filtrate is placed in a rotary evaporator and evacuated to remove solvent. The residue is an oil which weighs 9.3 g (0.03 mole) for a 97 percent yield is identified as DCTCS. Analysis of this residue by GLC shows that it contains ~96 percent of the aforementioned styrene and less than 4 percent of the diene.

Example 4

Dehydrohalogenation with $AlCl_3$/Alumina

A supported catalyst is prepared by slurrying 0.4 g of $AlCl_3$ and 4.5 g of alumina (~150 micrometers) in 20 ml of $CCl_4$ and then stirring the slurry at ~70°C for one hour. To this slurry at 70°C is added 250 g of a mixture of 125 g of DCTCB and 125 g of $CCl_4$ in less than 10 minutes. The resulting mixture is heated to 80°C and maintained there for 4 hours. During this time, the reaction mixture is stirred to maintain the supported catalyst dispersed therein. The reaction mixture is then filtered to remove solid catalyst. The resulting filtrate is evaporated to remove solvent. The remaining oily residue weighs 110 g (0.355 mole) indicating a yield of ~97 percent of DCTCS. Analysis of this residue by GLC shows that it contains ~97.6 percent of DCTCS and 2.8 percent of diene.

Example 5

Dehydrohalogenation with $TiCl_4$/Alumina

A supported catalyst is prepared by slurrying 0.45 g of $TiCl_4$ and 4.5 g of the alumina used in Example 2 in 20 ml of $CCl_4$ and then stirring the slurry at 70°C for one hour. To this slurry at 80°C is added 150 g of a mixture of 75 g of DCTCB and 75 g of $CCl_4$ in less than 10 minutes. The resulting mixture is heated to 80°C and maintained there for 4 hours. During this time, the reaction mixture is stirred to maintain the supported catalyst uniformly dispersed therein. The reaction mixture is then filtered to remove solid catalyst. The resulting filtrate is evaporated to remove solvent. The remaining oily residue weighs 65.5 g (0.211 mole) indicating a yield of ~96.5 percent of DCTCS. Analysis of this residue by GLC shows that it contains 95.6 percent of DCTCS and 3.8 percent of the diene.

Example 6

Dehydrohalogenation with $SnCl_4$/Alumina

A supported catalyst is prepared by slurrying 0.3 g of $SnCl_4$ and 3.0 g of the alumina used in Example 2 in 10 ml of $CCl_4$ and then stirring the slurry at 70°C for one hour. DCTCB is then dehydrohalogenated using this supported catalyst according to the procedure of Example 3. Analysis of the resulting dehydrohalogenated product shows that it contains 95.7 percent of DCTCS and 3.4 percent of the diene.

Example 7

Dehydrohalogenation with $SbCl_5$/Alumina

A supported catalyst is prepared by slurrying 0.2 g of $SbCl_5$ and 2.0 g of alumina (~150 micrometers) in 20 ml of $CCl_4$ and then stirring the slurry at ~70°C for one hour.

DCTCB is then dehydrohalogenated using this supported catalyst according to the

procedure of Example 3. Analysis of the dehydrohalogenated product shows that it contains 95.1 percent of DCTCS and 2.7 percent of the diene.

## Claims

1. A process for the dehydrohalogenation of a (polyhaloalkyl)arene wherein in the polyhaloalkyl one halogen is a benzylic halogen and at least one halogen is a non-benzylic halogen which process comprises contacting the (polyhaloalkyl)arene with a catalytic amount of a suitably active Lewis acid which is either (i) unsupported and is a halide of titanium which has been treated with water or a halide of antimony, or (ii) deposited upon an inert solid particulate support, the reaction being carried out at a temperature of below 100°C, to form a (haloalkenyl)arene.

2. A process as claimed in claim 1 wherein the (polyhaloalkyl)arene employed is represented by the formula:

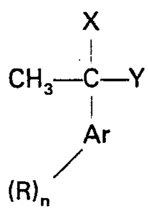

wherein Ar is arene, each R is individually halo, nitro or an inert monovalent, organic radical, X is halo, Y is haloalkyl or substituted haloalkyl wherein alkyl has 2 or 3 carbons and the non-halogen substituent or substituents are nitro or alkoxy; and n is 0 or a whole number from 1 to the maximum number of remaining available ring positions on Ar and the (haloalkenyl)arene is represented by the formula:

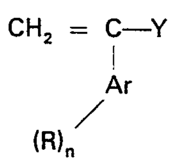

in which Y, Ar, R and n are as defined above.

3. A process as claimed in claim 2 wherein each R is individually halo, nitro, alkoxy, alkyl, haloalkyl, aryl or haloaryl, Y is polyhaloalkyl and n is 0, 1 or 2.

4. A process as claimed in claim 2 wherein the (polyhaloalkyl)arene is a (polyhaloalkyl)-benzene represented by the structural formula:

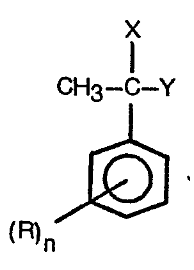

wherein X is chloro or bromo; R is Cl, Br, F, $-NO_2$, alkyl having 1 to 4 carbons, or alkoxy having 1 to 4 carbons; Y is haloalkyl represented by the formula:

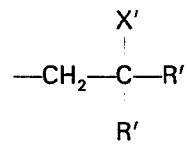

wherein X' is Cl or Br, each R' is individually H, Cl or Br; and n is 0, 1 or 2.

5. A process as claimed in any one of the preceding claims wherein the Lewis acid is $SbCl_5$ or $TiCl_4$ combined with from 0.25 to 1.75 moles of water per mole of $TiCl_4$.

6. A process as claimed in claim 5 wherein the (polyhaloalkyl)benzene is 1,3-dichloro-5-(1,3,3,3-tetrachloro-1-methylpropyl)benzene and the dehydrohalogenation is carried out in the presence of from 1.5 to 5 weight percent of $TiCl_4$ based on the weight of the (polyhaloalkyl)benzene and from 0.25 to 1.75 moles of water per mole of $TiCl_4$ at a reaction temperature in the range of from 25° to 80°C.

7. A process as claimed in any one of claims 1 to 4 wherein the Lewis acid is $AlCl_3$, $SbCl_5$, $TiCl_4$ or $SnCl_4$ which is deposited on alumina as the inert solid particulate support.

8. A process as claimed in claim 7 wherein the (polyhaloalkyl)benzene is 1,3-dichloro-5-(1,3,3,3-tetrachloro-1-methylpropyl)benzene and the dehydrohalogenation is carried out in the presence of from 0.1 to 10 weight percent of $AlCl_3$ and from 1 to 100 weight percent of alumina, both percentages being based on the weight of (polyhaloalkyl)benzene, at a reaction temperature in the range of from 25° to 80°C, the alumina having an average particle diameter in the range of from 100 to 400 micrometers.

## Patentansprüche

1. Verfahren zur Dehydrohalogenierung eines (Polyhalogenalkyl)arens, worin im Polyhalogenalkyl ein Halogenein benzylisches Halogen ist und mindestens ein Halogen ein nichtbenzylisches Halogen ist, dadurch gekennzeichnet, daß man das (Polyhalogenalkyl)aren mit einer katalytischen Menge einer geeigneten aktiven Lewis-Säure in Kontakt bringt, die entweder (i) trägerlos ist und ein mit Wasser behandeltes Titanhalogenid oder ein Antimonhalogenid ist, oder (ii) sich auf einem inerten festen teilchenförmigen Träger befindet, und die Reaktion zur Bildung eines (Halogenalkenyl)-arens bei einer Temperatur unterhalb 100°C durchführt.

2. Verfahren nach Anspruch 1, dadurch gekennzeichnet, daß das verwendete (Polyhalogenalkyl)aren durch die Formel

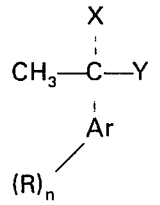

dargestellt wird, worin Ar Aren bedeutet, jedes R für sich Halogen, Nitro oder ein inertes monovalentes, organisches Radikal ist, X Halogen bedeutet, Y Halogenalkyl oder substituiertes Halogenalkyl ist, worin Alkyl 2 oder 3 Kohlenstoffatome besitzt und der nicht Halogen enthaltende Substituent oder Substituenten Nitro oder Alkoxy sind; und n 0 oder eine ganze Zahl von 1 bis zur maximalen Zahl der verbleibenden verfügbaren Ringpositionen an Ar ist, und das (Halogenalkenyl)aren durch die Formel

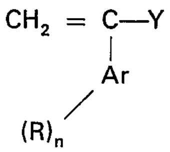

dargestellt wird, worin Y, Ar, R und n die oben angegebene Bedeutung besitzen.

3. Verfahren nach Anspruch 2, dadurch gekennzeichnet, daß jedes R für sich Halogen, Nitro, Alkoxy, Alkyl, Halogenalkyl, Aryl oder Halogenaryl bedeutet, Y ein Polyhalogenalkyl ist und n 0, 1 oder 2 ist.

4. Verfahren nach Anspruch 2, dadurch gekennzeichnet, daß das (Polyhalogenalkyl)aren ein (Polyhalogenalkyl)benzol ist, dargestellt durch die Strukturformel

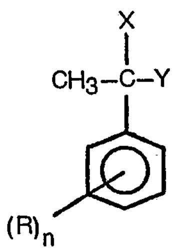

worin X Chlor oder Brom ist; R, Cl, Br, F, $-NO_2$, Alkyl mit 1 bis 4 Kohlenstoffatomen, oder Alkoxy mit 1 bis 4 Kohlenstoffatomen ist; Y eine durch die Formel

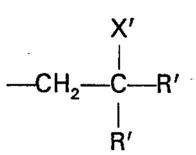

dargestelltes Halogenalkyl ist, worin X' Chlor oder Brom ist, jedes R' für sich H, Cl oder Br ist; und n 0, 1 oder 2 ist.

5. Verfahren nach einem der vorhergehenden Ansprüche, dadurch gekennzeichnet, daß die Lewis-Säure $SbCl_5$ oder ein mit 0,25 bis 1,75 Mol Wasser pro Mol $TiCl_4$ kombiniertes $TiCl_4$ ist.

6. Verfahren nach Anspruch 5, dadurch gekennzeichnet, daß das (Polyhalogenalkyl)-benzol 1,3-Dichlor-5-(1,3,3,3-tetrachlor-1-methylpropyl)benzol ist und die Dehydrohalogenierung in Gegenwart von 1,5 bis 5 Gew.-% $TiCl_4$, bezogen auf das Gewicht des (Polyhalogenalkyl)-benzols und von 0,25 bis 1,75 Mol Wasser pro Mol $TiCl_4$ bei einer Reaktionstemperatur im Bereich von 25 bis 80°C durchgeführt wird.

7. Verfahren nach einem der Ansprüche 1 bis 4, dadurch gekennzeichnet, daß die Lewis-Säure $AlCl_3$, $SbCl_5$, $TiCl_4$ oder $SnCl_4$ ist, das auf Aluminiumoxid als inerter fester teilchenförmiger Träger abgelagert ist.

8. Verfahren nach Anspruch 7, dadurch gekennzeichnet, daß das (Polyhalogenalkyl)-benzol 1,3-Dichlor-5-(1,3,3,3-tetrachlor-1-methylpropyl)-benzol ist und die Dehydrohalogenierung in Gegenwart von 0,1 bis 10 Gew.-% $AlCl_3$ und von 1 bis 100 Gew.-% Aluminiumoxid durchgeführt wird, wobei sich beide Prozentangaben auf das Gewicht von (Polyhalogenalkyl)-benzol beziehen, und bei einer Reaktionstemperatur im Bereich von 25 bis 80°C, wobei das Aluminiumoxid einen mittleren Teilchendurchmesser im Bereich von 100 bis 400 $\mu$m besitzt.

**Revendications**

1. Procédé pour la déshydrohalogénation d'un (polyhalogénoalcoyl)arène dans le groupe polyhalogénoalcoyle duquel un halogène est un halogène benzylique et au moins un halogène est un halogène non benzylique, procédé qui consiste à mettre en contact le (polyhalogénoalcoyle)arène avec une quantité catalytique d'un acide de Lewis convenablement actif qui est soit (i) non déposé sur support et qui est un halogénure de titane qui a été traité avec de l'eau ou bien un halogénure d'antimoine, soit (ii) déposé sur un support particulaire solide inerte, la réaction étant effectuée à une température inférieure à 100°C pour former un (halogénoalcényl)arène.

2. Procédé selon la revendication 1, dans lequel le (polyhalogénoalcoyl)arène utilisé est représenté par la formule:

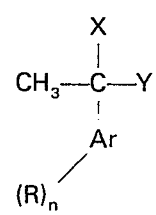

dans laquelle Ar désigne l'arène, chaque R est individuellement un groupe halogéno, nitro ou un radical organique monovalent, inerte; X est un halogène; Y est un groupe halogéno, alcoyle ou halogénoalcoyle substitué dans lequel le groupe alcoyle a 2 ou 3 atomes de carbone, et

le ou les substituant(s) non halogéné(s) est ou sont un ou des groupe(s) nitro ou alcoxy; et n est 0 ou un nombre entier de 1 jusqu'à un nombre maximum de positions sur le noyau restant disponsibles sur Ar et l'(halogénoalcényl)arène est représenté par la formule:

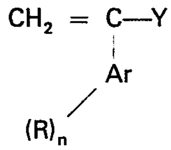

dans laquelle Y, Ar, R et n ont les définitions données ci-dessus.

3. Procédé selon la revendication 2, dans lequel chaque R est individuellement un groupe halogéno, nitro, alcoxy, alcoyle, halogénoalcoyle, aryle ou halogénoaryle; Y est un groupe polyhalogénoalcoyle, et n est 0, 1 ou 2.

4. Procédé selon la revendication 2, dans lequel le (polyhalogénoalcoyle)arène est un (polyhalogénoalcoyl)benzène représenté par la formule structurelle:

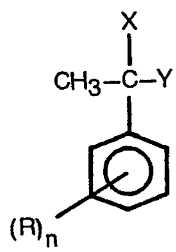

dans laquelle X est du chlore ou du brome; R est Cl, Br, F, $—NO_2$, un groupe alcoyle ayant 1 à 4 carbone, ou alcoxy ayant 1 à 4 carbones; Y est un groupe halogénoalcoyle représenté par la formule:

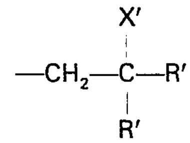

dans laquelle X' est Cl ou Br, chaque R' est individuellement H, Cl ou Br, et n est 0, 1 ou 2.

5. Procédé selon l'une quelconque des revendications précédentes, dans lequel l'acide de Lewis est $SbCl_5$ ou $TiCl_4$ combiné avec 0,25 à 1,75 mole d'eau par mole de $TiCl_4$.

6. Procédé selon la revendication 5, dans lequel le (polyhalogénoalcoyl)benzène est le 1,3-dichloro-5-(1,3,3,3-tétrachloro-1-méthyl-propyl)benzène et où la déshydrohalogénation est effectuée en présence de 1,5 à 5% en poids de $TiCl_4$, rapporté au poids du (polyhalogénoalcoyl)benzène, et de 0,25 à 1,75 mole d'eau par mole de $TiCl_4$, à une température de réaction comprise entre 25° et 80°C.

7. Procédé selon l'une quelconque des revendications 1 à 4, dans lequel l'acide de Lewis est $AlCl_3$, $SbCl_5$, $TiCl_4$ ou $SnCl_4$ qui est déposé sur de l'alumine comme support solide, particulaire, inerte.

8. Procédé selon la revendication 7, dans lequel le (polyhalogénoalcoyl)benzène est le 1,3-dichloro-5-(1,3,3,3-tétrachloro-1-méthyl-propyl)benzène et où la déshydrohalogénation est effectuée en présence de 0,1 à 10% en poids de $AlCl_3$ et de 1 à 100% en poids d'alumine, ces deux pourcentages étant calculés sur le poids du (polyhalogénoalcoyl)benzène, à une température de réaction comprise entre 25° et 80°C, l'alumine ayant un diamètre particulaire moyen compris entre 100 et 400 micromètres.